# EUROPEAN PATENT APPLICATION

(11) **EP 2 949 360 A1**
(43) Date of publication of application: **02.12.2015**
(21) Application number: 13873054.4
(22) Date of filing: 22.01.2013
(51) Int. Cl.: A61N 5/10

(54) **PARTICLE BEAM IRRADIATION DEVICE, AND PARTICLE BEAM THERAPY DEVICE PROVIDED THEREWITH**

(71) Applicant: Mitsubishi Electric Corporation, Tokyo 100-8310 (JP)
(72) Inventor: PU, Yuehu, Tokyo 100-8310 (JP); IKEDA, Masahiro, Tokyo 100-8310 (JP); HONDA, Taizo, Tokyo 100-8310 (JP)
(74) Representative: Sajda, Wolf E.
(86) International application number: PCT/JP2013/051133
(87) International publication number: WO 2014/115237

(57) **Abstract**

A particle beam irradiation system comprising a scanning electromagnet (2) for a particle beam to scan an irradiation object, a scanning information storage section (4) for storing scanning position information regarding a plurality of scanning positions in a case where a particle beam scans an irradiation object and scanning order information which is order for scanning a plurality of scanning positions, and a scanning electromagnet control section (8) for controlling a scanning electromagnet based on scanning position information and scanning order information which is stored in the scanning information storage section, wherein the scanning position information which is stored in the scanning information storage section includes a part whose scanning position information is same as the scanning position information of adjacent order.

## Description

### Technical Field

This invention relates to a particle beam irradiation system which is used for irradiating a particle beam in accordance with a three-dimensional shape of an affected part in a particle beam therapy system which performs therapy by irradiating an affected part such as a tumor with a particle beam.

### Background Art

In therapy by using particle beams, a high energy particle beam such as a proton or a carbon ion beam, etc. which is accelerated up to approximately 70 % of light speed is used. When the above-mentioned high energy particle beam is irradiated into a body, the above-mentioned high energy particle beam has the following features. Firstly, most of particles which are irradiated stop at a position whose depth is proportional to approximately 1.7 squared of particle energy.

Secondly, energy density (called dose) which is given to a path where a particle beam passes before it stops in the body has a maximum value at a positon where a particle stops. A specific depth dose distribution curve which is formed in accordance with a path where a particle beam passes is called a Bragg curve, and a position at which a dose value is maximum is called a Bragg peak.

According to a three-dimensional particle beam irradiation system, while a position of this Bragg peak is scanned in accordance with a three-dimensional shape of a tumor and peak dose at each scanning position is adjusted, in a tumor region, a target which is determined by an image diagnosis in advance, it is designed to form a predetermined three-dimensional dose distribution.

A scanning of a particle beam at a stop position includes a scanning of a particle beam at a lateral direction which is substantially perpendicular to an irradiation direction of a particle beam (X-direction, Y-direction) and a scanning of a particle beam at a depth direction (Z-direction) which is an irradiation direction of a particle beam.

A scanning at a lateral direction includes a method in which a patient is moved with respect to a particle beam, and a method in which a position of a particle beam is moved by using an electromagnet, etc., and a method using an electromagnet is generally used. Regarding a scanning at a depth direction, changing particle energy is only method. Regarding method of changing energy, there are two methods.

One of the methods is a method of changing particle energy by using an accelerator, and another method is a method in which an energy changing device, which is called a range shifter (including an Energy Selection System which is a device for energy changing and analysis) which is provided at a beam transport system or an irradiation system, is used. In many cases, a method using a range shifter is used.

Broadly, there are two basic methods for scanning a particle beam perpendicular to a travelling direction of a particle beam, that is, in a lateral direction. One of the methods is a spot scanning irradiation method, that is, when a particle beam is irradiated and irradiation dose at a predetermined irradiation position reaches a planned value, particle beam intensity is weakened once (generally, particle beam intensity is made to be zero), a current value of a scanning electromagnet is changed so as to make a particle beam irradiate at next irradiation position, particle beam strength is increased again (or a particle beam is irradiated form an accelerator again), and then a particle beam is irradiated.

For example, an example of the above-mentioned is shown in Non-Patent Document 1. Another method is a hybrid scanning irradiation method. According to a hybrid scanning irradiation method, basic procedure for irradiating planned amount of particle beam at each planned position is the same, however, when a position of a particle beam is moved to next irradiation position, a particle beam is scanned without stopping a particle beam, that is, while irradiating a particle beam. For example, an example of the above-mentioned is disclosed in Non-Patent Document 2.

Further, there is a method for forming a predetermined three dimensional dose distribution in an irradiation region by performing a two-dimensional scanning of a particle beam in a lateral direction so as to form a lateral direction dose distribution having a predetermined distribution, by changing particle energy in a depth direction so as to form a plurality of lateral direction dose distributions having a predetermined layered distribution at depth positions whose irradiation regions are different, and by overlapping the above-mentioned layered dose distributions.

In this regard, in many cases, each layered dose distribution is made to be a uniform distribution. In order to do the above-mentioned, planned irradiation particle amount at an irradiation position in each layer which is determined in advance is calculated in a treatment plan.

In any of the above-mentioned methods, in order to form a predetermined two-dimensional or three-dimensional dose distribution, in a treatment plan, etc., planned irradiation dose at each of planned irradiation position is determined.

### Prior Art References

### Patent Documents

Patent Document 1 JP 3 874 766 B
Patent Document 2 US 2006/0231775 A

### Non-patent documents

Non-patent document 1 T. Inaniwa et al., Medical Physics 34(2007)3302
Non-patent document 2 J.H. Kang et al., "Demonstration of scan path optimization in proton therapy" Medical Physics 34(9)2007, pages 3457 to 3464

### Disclosure of the Invention

### Problems to be Solved by the Invention

In a particle beam scanning irradiation system which is mentioned in Patent Document 1, it is necessary to store the number of planned irradiating particles (irradiation dose) which is irradiated by scanning irradiation at each spot (position) in a predetermined memory of an irradiation system as a preset value and at the same time, it is also necessary to store information corresponding to a position of each spot (for example, a pair of excitation currents of an electromagnet (IXi, IYi),I = 1,2,3...Nspot, etc.) in a predetermined memory of an irradiation system.

Then, in performing irradiation, irradiation dose at each irradiation position is counted by a beam monitor, etc., when the count value reaches a preset value corresponding to the position, it is judged such that irradiation at the position is completed, the system is configured to perform an irradiation at next irradiation position. Conventional irradiation systems in a therapy system of a particle beam or an X-ray are constructed by a method in which the preset value and the count value are collated.

As above mentioned, in a particle beam irradiation system in conventional particle beam therapy systems, when a particle beam is irradiated at each irradiation position, it is absolutely necessary to perform an operation for collating a count value of particle beam, that is an amount of an irradiated particle beam at the position, which is counted by a beam monitor, with a preset value. In general, it requires several tens microseconds to collate a count value with a preset value.

When it is intended to perform irradiation at all irradiation positions in a short time by strengthening a particle beam, in some cases, an operation for collating a count value with a preset value is an obstacle for speeding up of operation. Therefore in traditional irradiation systems, it is still difficult to realize an extremely high dose rate (dose which can be irradiated per hour).

The present invention aims to solve the above-mentioned problems of conventional particle beam irradiation systems, and aims to provide a particle beam therapy system and a particle beam irradiation system whose dose rate is high, that is, a beam current of a particle beam therapy system can be extensively utilized and irradiation can be completed within a short time.

### Means for Solving the Problems

A particle beam irradiation system according to the present invention comprises
- a scanning electromagnet for a particle beam to scan an irradiation object,
- a scanning information storage section which stores scanning position information regarding a plurality of scanning positions in a case where a particle beam scans the irradiation object and scanning order information regarding order of scanning a plurality of scanning positions and
- a scanning electromagnet control section for controlling a scanning electromagnet based on the scanning position information and the scanning order information which is stored in the scanning information storage section, wherein the scanning position information which is stored in the scanning information storage section includes a part whose scanning position information is same as the scanning position information of adjacent order.

### Advantage of the Invention

According to the particle beam irradiation system of the present invention, as the scanning position information includes a part whose scanning position information is same as that of a part of adjacent order, a particle beam irradiation system, which has high dose rate and by which irradiation can be completed in a short time, can be provided.

### Brief Description of the Drawings

- FIG. 1: is a block diagram showing a schematic configuration of a particle beam irradiation system according to an Embodiment of the present invention.
- FIG. 2: is a table showing an example of a scanning position and planned irradiation dose which is planned in a treatment planning device.
- FIG. 3: is a table showing an example of scanning information which is stored in a scanning information storage section of a particle beam irradiation system according to an Embodiment of the present invention.
- FIG. 4: is a conceptual diagram for describing an operation of a particle beam irradiation system according to an Embodiment of the present invention.
- FIG. 5: is a block diagram showing another schematic configuration of a particle beam irradiation system according to an Embodiment of the present invention.

### Embodiment for Carrying Out the Invention

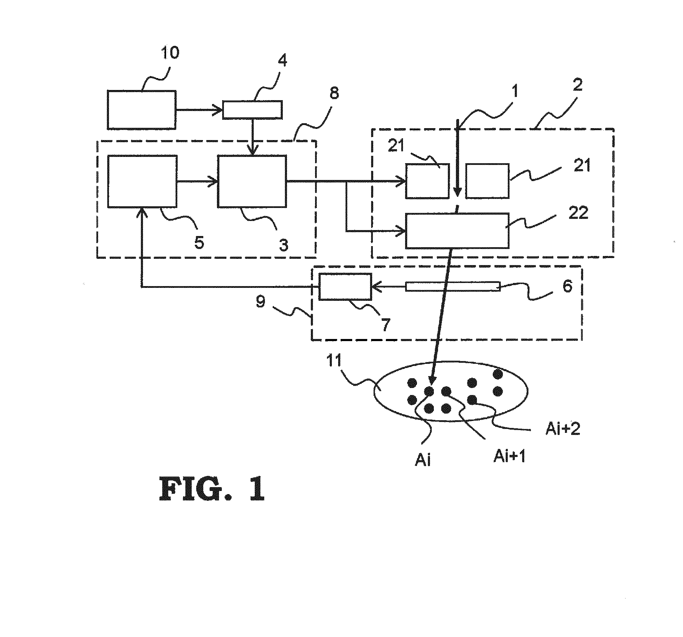

FIG. 1 is a block diagram showing a schematic configuration of a particle beam irradiation system according to Embodiment of the present invention. The particle beam irradiation system is configured for a particle beam 1 which is generated by a particle beam accelerator which is not shown in the figure and is transported by a particle beam transport system which is not shown in the figure to scan an irradiation object 11 such an affected part of a patient by an X-direction scanning electromagnet 21 which deflects the particle beam 1 to one direction and a Y-direction scanning electromagnet 22 which deflects the particle beam 1 to another direction which is perpendicular to the one direction. Here, combination of the X-direction scanning electromagnet 21 and the Y-direction scanning electromagnet 22 refers to as a scanning electromagnet 2.

The scanning electromagnet 2 is driven by a scanning electromagnet driving power source 3 which is controlled by a scanning electromagnet power source control section 5. In a scanning information storage section 4, scanning position information regarding a plurality of scanning positions and scanning order information regarding scanning order is stored as scanning information.

The scanning electromagnet power source control section 5 receives a monitor pulse which is output by a monitor pulse generating section 7 corresponding to particle beam dose which is detected by a particle beam monitor sensor 6 and controls the scanning electromagnet driving power source 3, and the scanning electromagnet driving power source 3 drives the scanning electromagnet 2 based on information which is stored in the scanning information storage section 4.

Here, a combination of the scanning electromagnet driving power source 3 and the scanning electromagnet power source control section 5 refers to a scanning electromagnet control section 8, and a combination of the particle beam monitor sensor 6 and the monitor generating section 7 refers to a particle beam monitor 9.

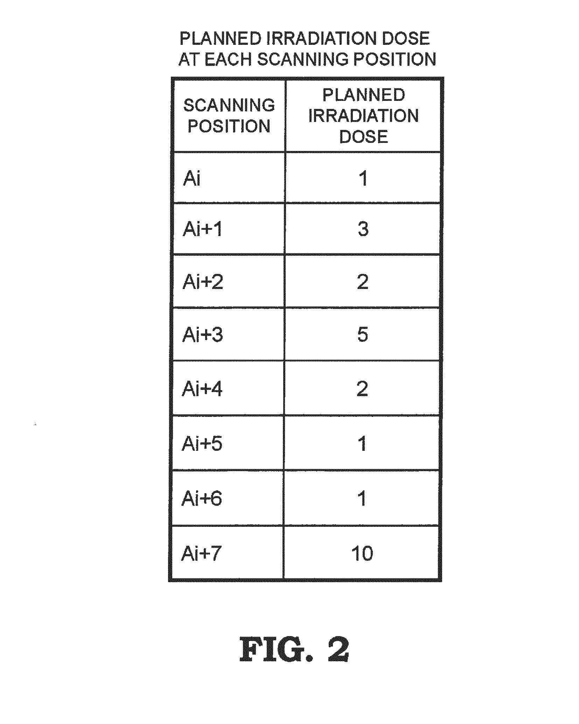

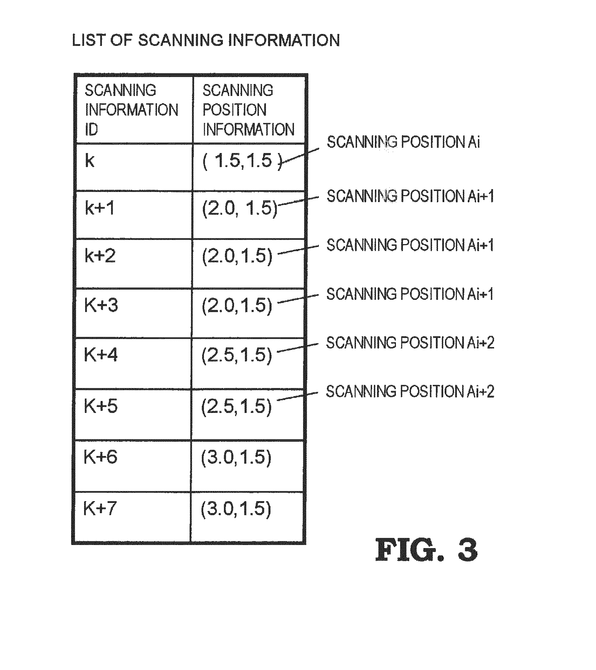

FIG. 2 is a table showing a list of example of planned irradiation dose with respect to each scanning position in a therapy irradiation system according to Embodiment of the present invention. FIG. 3 is a table showing an example of content of scanning information which is stored in the scanning information storage section 4 of a particle beam irradiation system according to Embodiment of the present invention.

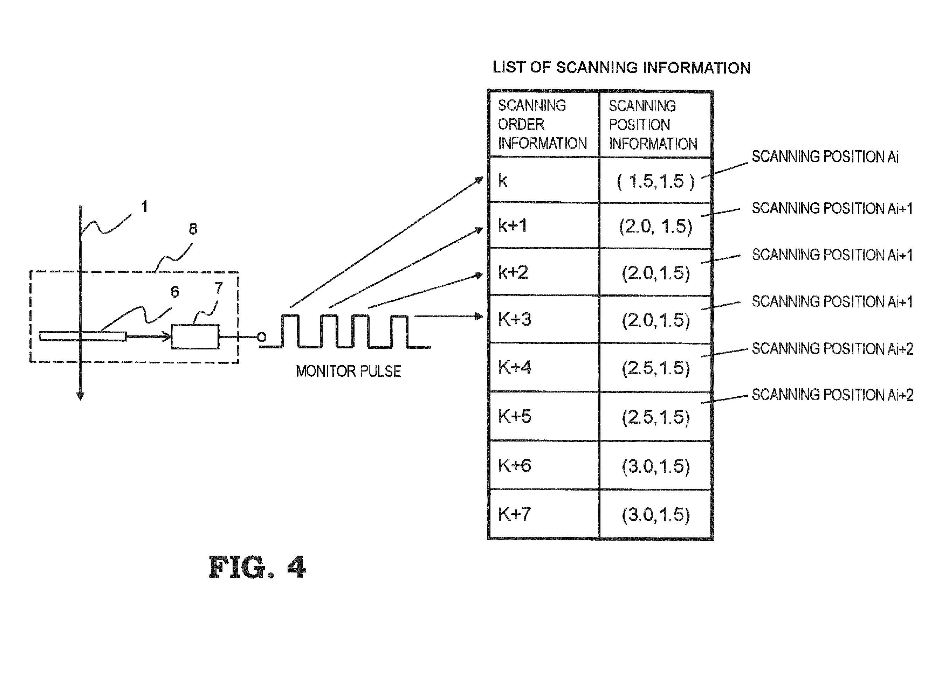

FIG. 4 shows an example of a monitor pulse and its train which is output by the monitor generating section 7 corresponding to a fixed amount of electric charge (for example, Q0) which is input from the particle beam monitor sensor 6, and scanning information which is stored in the scanning information storage section 4.

Hereinafter, referring FIGS. 1 to 4, an operation of a particle beam irradiation system according to Embodiment of the present invention will be described. At first, a treatment plan is made based on CT data, etc. of a patient, in an irradiation object 11 in FIG. 1, a plurality of all irradiation positons (in FIG. 1, for describing hereinafter, marks are given to three irradiation positions, that is, irradiation position Ai, irradiation position Ai+1 and irradiation position Ai+2), order of scanning irradiation position and planned irradiation doze at each irradiation position is determined.

Position information corresponding to an irradiation position includes a position coordinate in a lateral direction and a depth position in an irradiation object. Position information in a lateral direction is basically defined by an excitation current IX of the X-direction electromagnet 21 and an excitation current IY of the Y-direction electromagnet 22 of the scanning electromagnet 2, etc.

Depth position information can be defined by particle energy of a particle beam which is irradiated to the irradiation object 11. This is because such that a depth position where a particle beam stops in the body, that is, a depth position where a particle beam is irradiated onto is determined by energy of a particle beam.

There are many methods for irradiating a particle beam to the irradiation object 11 including a scanning irradiation method, a uniform scanning method, a simulated scattering, etc. However, in any case, a plurality of irradiation positions, order of scanning an irradiation position, and an irradiation dose (which can be simply considered as the number of particles of a particle beam which is irradiated) which is irradiated at each irradiation position are determined by a treatment planning device 10. A difference between each irradiation method includes the number of irradiation positions, a method to apply a particle beam, irradiation order, an irradiation dose at each irradiation position, etc.

Hereinafter, following description will be made by using a scanning irradiation method as an example, it is needless to say such that the present invention can be applied to other irradiation method in which irradiation is performed by designating a plurality of irradiation positions, order of scanning irradiation positions, and irradiation dose at each irradiation position.

In a scanning irradiation method, an operation, in which a particle beam is stayed at each irradiation position to irradiate a particle beam so as for irradiation dose to reach planned irradiation, after that, the particle beam is moved to next irradiation position to irradiate the irradiation position so as for irradiation dose to reach planned irradiation dose, is repeated so as to irradiate the particle beam at all irradiation positions which are determined by the treatment planning device 10.

In performing a particle beam therapy, in some cases, the number of irradiation position amounts to thousands. Hereinafter, an operation of a particle beam irradiation system according to Embodiment of the present invention will be described using an example in which several irradiation positions in which a depth direction position at which irradiation is performed in order is same.

FIG. 2 is a table showing an example of irradiation dose which is planned at each scanning position by the treatment planning device 10, that is, planned irradiation dose. As shown in FIG. 2, planned irradiation dose to be irradiated at each scanning position is determined by a treatment plan, for example, planned irradiation dose at scanning position Ai is one unit, planned irradiation dose at scanning position Ai+1 is three units, and planned irradiation dose at scanning position Ai+2 is two units.

Then, based on a planned irradiation dose at each scanning position shown in a table of FIG. 2 for example, in the treatment planning device 10, a list of scanning information shown in FIG. 3 is made and stored in the scanning storage section 4.

As shown in FIG. 3, scanning information which is stored in the scanning information storage section 4 includes scanning position information which shows a scanning position and scanning information ID, that is, scanning order information which shows scanning order at the scanning position.

For example, as scanning position information whose scanning order is kth, a pair of information of X and Y (1.5, 1.5) corresponding to scanning position Ai, is stored. The (1.5, 1.5) indicates (IX_i,IY_i), that is a pair of an excitation current IX_i of the X-direction scanning electromagnet 21 and an excitation current IY_i of the Y-direction scanning electromagnet 22 corresponding to scanning position Ai of the scanning electromagnet 2.

That is, when an excitation current of the X-direction scanning electromagnet 21 is set to be 1.5, and an excitation current of the Y-direction scanning electromagnet 22 is set to be 1.5, a particle beam 1 irradiates scanning position Ai.

Subsequently, in scanning order (k+1)th, (k+2)th and (k+3)th of scanning position information, as scanning position information corresponding to scanning position Ai+1, a pair of an excitation current IX_i+1 of an X-direction electromagnet and an excitation current IY_i+1 of a Y-direction electromagnet, that is, (2.0,1.5) is stored.

Since planned irradiation dose at scanning irradiation position Ai shown in an example in FIG. 2 is 1, according to a scanning information list shown in FIG. 3, scanning position information corresponding to scanning position Ai is only one, kth.

Since planned irradiation dose at scanning position Ai+1 is 3, as successive three pieces of scanning position information, that is, (k+1)th, (k+2)th and (k+3)th, the same pair of excitation currents corresponding to scanning position Ai+1, that is, (2.0,1.5) is stored.

Similarly, regarding scanning position Ai+2, planned irradiation dose is 2, therefore, as scanning position information regarding two pieces of successive scanning order, that is, scanning position information whose scanning order is (k+4)th and (k+5)th, the same pair of excitation currents corresponding to scanning position Ai+2, that is, (2.5,1.5) is stored.

As above-mentioned, in a particle beam irradiation system of the present invention, scanning position information which is stored in the scanning information storage section 4 includes a part whose scanning position information is same as that of adjacent order.

Then, in performing irradiation, whenever the scanning electromagnet control section 8 receives a monitor pulse, in accordance with a order of scanning information list shown in FIG. 3 which is stored in the scanning information storage 4, the scanning electromagnet 2 is excited so as to scan a particle beam.

FIG. 4 specifically shows how the scanning electromagnet 2 is excited in scanning a particle beam. FIG. 4 shows an example in a case where a particle beam is scanned at irradiation position Ai, irradiation position Ai+1 and irradiation position Ai+2 in order, during irradiation.

While the particle beam 1 is irradiated, the particle beam 1 passes through the particle beam monitor sensor 6 so as to irradiate to the irradiation object 11. When the particle beam 1 passes through the particle beam monitor sensor 6, an ionization current which is proportional to the number of particles which pass through the particle beam monitor sensor 6 is generated.

The ionization current is integrated by the monitor pulse generating section 7, and when an integrated value reaches a fixed amount of an electric charge Q0, the monitor pulse generating section 7 outputs one monitor pulse. By appropriately setting a gain of the monitor pulse generating section 7, a monitor pulse can be output for every necessary value of Q0.

In a case where a particle beam irradiation system according to Embodiment of the present invention is used as a particle beam therapy system, an example in which a value of Q0 is in a range between 0.01 pC and 100 pC is typical, however, this is not limited thereto.

A monitor pulse which is output by the monitor pulse generating section 7, that is, the particle beam monitor 9 is input to the scanning electromagnet power source control section 5 of the scanning electromagnet control section 8 shown in FIG. 1. Every time when the scanning electromagnet power source control section 5 receives a monitor pulse, the scanning electromagnet power source control section 5 transmits a command signal to the scanning electromagnet driving power source 3.

The scanning electromagnet driving power source 3 successively renews an excitation current value to an excitation current value corresponding to an address (k, k+1, k+2, k+3,...) which is adjacent in the scanning information list which is stored in the scanning information storage section 4, and successively renews an excitation amount of the scanning electromagnet 2 so as for the particle beam 1 to scan the irradiation object 11.

As above-mentioned, a monitor pulse which is output by the particle beam monitor 9 is a kind of clock pulse, that is, a dose clock pulse, and for every dose clock pulse, according to an excitation current which is scanning position information in a scanning information list, the scanning electromagnet 2 is successively excited.

As a result, without directly counting an amount of an irradiation particle at each irradiation position, a particle beam whose amount is same as that of planned irradiation dose or that which is proportional to the planned irradiation dose can be irradiated at each irradiation position. As a result, in the irradiation object 11, a dose distribution according to treatment plan can be formed.

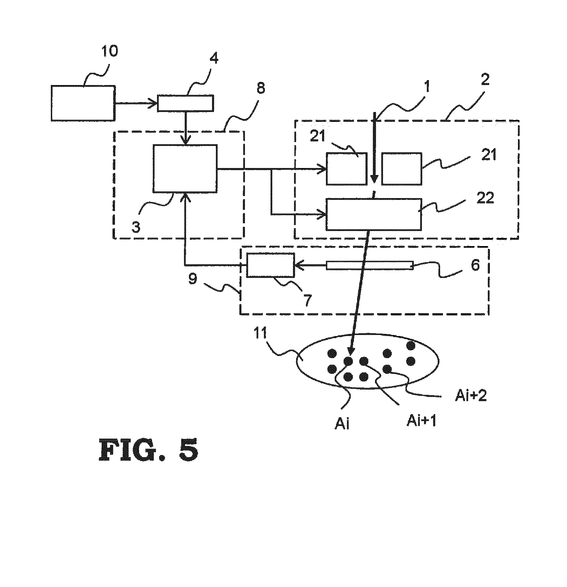

In the above-mentioned configuration, in addition to the scanning electromagnet driving power source 3, the scanning electromagnet power source control section 5 is provided, however, the configuration in which the scanning electromagnet power source control section 5 is not provided, and the scanning electromagnet driving power source 3 has a control function is also acceptable. The above-mentioned configuration is shown in FIG. 5. In this case, the scanning electromagnet driving power source 3 is the scanning electromagnet control section 8.

In the configuration of a particle beam irradiation system shown in FIG. 5, every time when the scanning electromagnet driving power source 3 receives a monitor pulse which is output by the particle beam monitor 9, scanning position information, that is, information regarding an excitation current of the X-direction scanning magnet 21 and an excitation current of the Y-direction scanning magnet 22 is sequentially fetched by the scanning electromagnet driving power source 3 itself from the scanning information storage section 4 so as to drive the scanning electromagnet 2.

Further, in the above, a case in which scanning position information which is stored in the scanning information storage section 4 is a pair of excitation currents of a scanning electromagnet is described as an example, however, scanning position information which is stored in the scanning information storage section 4 may be a coordinate position itself in which an isocentre is reference.

In this case, when the scanning electromagnet driving power source 3 has a correspondence table of coordinate positions and excitation currents of the scanning electromagnet 2, it is acceptable. Further, as scanning positon information, magnetic field strength is acceptable.

For example, it may be configured such that a magnetic field sensor measuring a magnetic field which is generated by the X-direction scanning electromagnet 21 and that which is generated by the Y-direction scanning electromagnet 22, respectively, is provided and the X-direction scanning electromagnet 21 and the Y-direction electromagnet 22 are driven so as for output of the magnetic field sensor to be magnetic field strength as scanning position information.

Further, in a case of configuration in which a particle beam is deflected by an electric field, that is, a particle beam is deflected by a scanning electrode, scanning positon information may be electric field strength or electrode voltages.

As above mentioned, in a particle beam irradiation system according to an Embodiment of the present invention, at first, planned irradiation dose at each irradiation position is reflected on scanning position information which is stored in the scanning information storage section 4. By using a monitor pulse which is output by the particle beam monitor 9, the scanning electromagnet driving power source 3 is directly driven by scanning position information which is stored in the scanning information storage section 4 so as to irradiate planned particle dose at each scanning position.

Consequently, unlike a conventional particle beam therapy system, it is not necessary to store a monitor preset value corresponding to planned irradiation dose at each irradiation position in a control device, further, it is not necessary to perform operation in which output of the monitor sensor 6 is compared with a preset value. Consequently, a particle beam can be scanned faster so as to irradiate an object. As a result, treatment time can be shortened.

Further, dose rate which is irradiation dose per time can be increased, when a target which is moved by respiration (a tumor) is irradiated, irradiation can be completed in a shorter time, and errors of dose caused by fluctuation of position can be decreased. As a result, a particle beam therapy system with higher accuracy can be provided.

Further, within the scope of the present invention, embodiment of the invention can be appropriately deformed or omitted.

### Remarks

- 1: particle beam
- 2: scanning electromagnet
- 3: scanning electromagnet driving power source
- 4: scanning information storage section
- 5: scanning electromagnet power source control section
- 6: particle beam monitor sensor
- 7: monitor pulse generating section
- 8: scanning electromagnet control section
- 9: particle beam monitor
- 10: treatment planning device
- 11: irradiation object

## Claims

1. A particle beam irradiation system comprising
- a scanning electromagnet for a particle beam to scan an irradiation object,
- a scanning information storage section which stores scanning position information regarding a plurality of scanning positions in a case where a particle beam scans the irradiation object and scanning order information regarding scanning order which is order for scanning the plurality of scanning positions and
- a scanning electromagnet control section for controlling the scanning electromagnet based on the scanning position information and the scanning order information which is stored in the scanning information storage section,
wherein the scanning position information which is stored in the scanning information storage section includes a part whose scanning position information is same as the scanning position information of adjacent order.

2. The particle beam irradiation system according to claim 1,
wherein a number of pieces of successive same scanning position information when the same scanning information is stored successively is proportional to planned irradiation dose of a particle beam at a scanning position corresponding to the same scanning position information.

3. The particle beam irradiation system according to claim 2,
further comprising a particle beam monitor which generates a monitor pulse every time when a predetermined amount of the particle beam passes;
wherein the scanning electromagnet control section fetches the scanning position information of the next order, which is stored in the scanning information storage section, every time when the monitor pulse is generated and controls the scanning electromagnet based on the scanning position information of the next order.

4. The particle beam irradiation system according to any one of claims 1 to 3,
wherein the scanning position information is an excitation current value of the scanning electromagnet corresponding to the scanning position.

5. The particle beam irradiation system according to any one of claims 1 to 3,
wherein the scanning position information is magnetic field strength, which is generated by the scanning electromagnet, corresponding to the scanning position.

6. A particle beam therapy system in which the particle beam irradiation system according to claim 2 or 3 is provided,
wherein a treatment planning device is provided determining the plurality of scanning positions, order of scanning the plurality of scanning positions, and planned irradiation dose of the particle beam at each of the scanning positions.
